(19) Europäisches Patentamt
European Patent Office
Office européen des brevets

(11) **EP 2 059 230 B1**

(12) **FASCICULE DE BREVET EUROPEEN**

(45) Date de publication et mention
de la délivrance du brevet:
**30.11.2016 Bulletin 2016/48**

(51) Int Cl.:
***A61K 8/97*** *(2006.01)*     ***A61Q 17/00*** *(2006.01)*
***A61K 8/64*** *(2006.01)*

(21) Numéro de dépôt: **07823783.1**

(22) Date de dépôt: **06.09.2007**

(86) Numéro de dépôt international:
**PCT/FR2007/051887**

(87) Numéro de publication internationale:
**WO 2008/029064 (13.03.2008 Gazette 2008/11)**

(54) **UTILISATION TOPIQUE D'UN EXTRAIT PEPTIDIQUE DE SOJA ET/OU DE BLE COMME AGENT PHOTOPROTECTEUR**

TOPISCHE VERWENDUNG EINES PEPTIDEXTRAKTS AUS DER SOJABOHNE UND/ODER WEIZEN ALS LICHTSCHUTZMITTEL

TOPICAL USE OF A PEPTIDE EXTRACT OF SOYBEAN AND/OR OF WHEAT AS PHOTOPROTECTIVE AGENT

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **06.09.2006 FR 0607851**
**06.09.2006 FR 0607852**

(43) Date de publication de la demande:
**20.05.2009 Bulletin 2009/21**

(73) Titulaire: **THOREL, Jean-Noël**
**75014 Paris (FR)**

(72) Inventeur: **THOREL, Jean-Noël**
**75014 Paris (FR)**

(74) Mandataire: **Cabinet Laurent & Charras**
**Le Contemporain**
**50 Chemin de la Bruyère**
**69574 Dardilly Cedex (FR)**

(56) Documents cités:
**EP-A- 1 618 871**     **EP-A- 1 815 843**
**WO-A-00/64472**     **WO-A-02/17862**
**WO-A-02/45666**     **WO-A-03/068184**
**DE-A1-102005 063 179**     **FR-A- 2 758 721**
**FR-A- 2 775 185**     **US-A- 5 571 503**
**US-B1- 6 365 656**

Remarques:
Le dossier contient des informations techniques présentées postérieurement au dépôt de la demande et ne figurant pas dans le présent fascicule.

**EP 2 059 230 B1**

**Description**

La présente invention se rapporte à une composition cosmétique et/ou dermatologique destinée à prévenir l'altération des cellules de la peau, des lèvres, des cheveux et/ou des muqueuses par les radiations lumineuses et plus généralement, à agir comme agent photoprotecteur.

La composition de la présente invention peut appartenir à la gamme des produits anti-solaires, mais également à la gamme des produits de soin, comme par exemple des laits pour le corps ou des crèmes de jour, des sticks à lèvres, ainsi qu'à la gamme des produits de maquillage ou de traitement capillaire.

En effet, pour l'ensemble de cette gamme de produits, on cherche à lutter contre les effets néfastes des UVA et des UVB sur les cellules de la peau, des lèvres, des cheveux et/ou des muqueuses.

En effet, même si les radiations lumineuses de longueur d'onde comprise entre 290 nm et 400 nm, correspondant aux UVA (de longueur d'onde comprise entre 320 nm et 400 nm) et aux UVB (de longueur d'onde comprise entre 290 nm et 320 nm), sont nécessaires au bon fonctionnement de l'organisme humain, elles provoquent aussi de nombreux effets négatifs. Les UVB sont ainsi plus particulièrement à l'origine d'érythèmes, de brûlures cutanées, tandis que les UVA sont à priori responsables de la formation de radicaux libres qui sont susceptibles d'induire une altération cutanée à long terme. Ces rayonnements UV doivent donc être filtrés.

De nombreuses compositions cosmétiques telles que, par exemple, produits anti-solaires ou encore crèmes de jours assurant la photoprotection (UVA et/ou UVB) de la peau ont été proposées à ce jour. Ces compositions se présentent assez souvent sous la forme d'une émulsion qui contient, à des concentrations diverses, un ou plusieurs filtres solaires. Il existe deux types de filtres solaires : d'une part les filtres organiques classiques lipophiles et/ou hydrophiles capables d'absorber sélectivement les rayonnements UV nocifs. Ils peuvent être introduits assez facilement dans les émulsions en les dispersant soit dans la phase huileuse, soit dans la phase aqueuse de l'émulsion (selon leur caractère lipophile ou hydrophile). Le type de filtres et leur quantité sont sélectionnés en fonction de l'indice de protection recherché.

Il existe d'autre part les filtres minéraux, qui réfléchissent la lumière, utilisés en plus ou à la place des filtres organiques. Il s'agit en particulier des oxydes métalliques comme par exemple le dioxyde de titane et l'oxyde de zinc, qui confèrent d'excellentes propriétés protectrices contre les UV et une très bonne tolérance cutanée.

Néanmoins, ces deux catégories de filtres solaires présentent certains inconvénients. Les filtres organiques doivent être utilisés en grande quantité pour obtenir de forts indices de protection et ils peuvent alors être responsables de problèmes de tolérance ; de plus, ils sont soupçonnés de possibles actions hormonales oestrogéniques, ce qui pourrait être biologiquement dangereux, si ces risques étaient confirmés, en particulier chez les jeunes garçons. Les filtres minéraux, quant à eux, posent des problèmes de galénique cosmétique. En effet, les produits de protection solaire les contenant se présentent souvent sous forme d'émulsions plus ou moins épaisses, difficiles à appliquer et à étaler, lourdes et poisseuses. En outre, à ces défauts s'ajoute, avec certains filtres minéraux comme le dioxyde de titane, un effet de blanchissement lors de l'étalement sur la peau.

Les documents US 5,571,503, FR 2 775 185, WO 03/068184, WO 00/64472, WO 02/17862, EP 1 618 871, WO 02/45666, US 6,365,656 et FR 2 758 721 décrivent des compositions complexes comprenant un extrait de blé et/ou de soja, et présentant éventuellement une activité protectrice vis-à-vis des UV, sans que celle-ci ne puisse être attribuée audit extrait.

Le document EP 1 815 843 décrit l'effet bénéfique sur la synthèse de collagène de l'association de deux extraits peptidiques de soja et d'un extrait peptidique de blé. Il est évoqué la possibilité de leur ajouter un autre composé stimulant la synthèse de collagène choisi dans une longue liste dans laquelle figure la créatine mais aussi le tripeptide Gly-His-Lys. Aucun effet direct sur la protection vis-à-vis des UVA/UVB lié à de telles associations n'est décrit ni même suggéré par ce document.

Le but de la présente invention est donc de surmonter ces inconvénients et de proposer une composition photoprotectrice destinée à prévenir l'altération des cellules de la peau, des lèvres, des cheveux et/ou des muqueuses par les radiations lumineuses du type UVA et UVB ne contenant pas ou très peu de filtres solaires.

Le Demandeur a constaté que de manière tout à fait surprenante, il était possible de lutter efficacement contre les effets nocifs à la fois des UVA et des UVB, en agissant biologiquement au niveau des cellules de la peau et non plus au moyen d'un filtre ou d'un écran anti UVA ou anti UVB. Avantageusement, cette action biologique peut être mise en oeuvre au moyen de substances biomimétiques, c'est-à-dire de substances présentant la structure et/ou exerçant la fonction ou l'activité biomécanique, biophysique, ou biologique de tout composant cutané, l'objectif étant d'adapter la réponse des cellules de la peau aux effets des rayonnements UVA et UVB.

En d'autres termes, le Demandeur a cherché à substituer tout ou partie des filtres solaires connus et désignés par la suite comme « filtre solaire externe », par des constituants assurant une action biologique au niveau des cellules de la peau, des lèvres, des cheveux et/ou des muqueuses permettant de protéger lesdites cellules contre les effets néfastes des UVA et UVB. Cette action biologique également désignée par la suite comme « protection interne » peut consister en :

- une protection de l'ADN,
- une diminution de l'immunosuppression,
- une action antiradicalaire etc....

Cette nouvelle approche a permis dans un premier temps au Demandeur de découvrir que les extraits peptidiques de soja ou de blé, lorsqu'ils étaient appliqués in vivo, sur la peau, permettaient de protéger biologiquement les cellules de la peau, des lèvres, des cheveux et/ou des muqueuses contre les effets néfastes des UVA et UVB.

Ainsi, la présente demande décrit l'utilisation topique d'un extrait peptidique de soja et/ou de blé comme photoprotecteur et plus précisément comme agent destiné à lutter contre l'altération des cellules de la peau, des lèvres, des cheveux et/ou des muqueuses provoquée par les UVA et les UVB, avantageusement dans ce second cas lorsque ces extraits sont associés à d'autres constituants assurant la « protection interne » des cellules et/ou des « filtres solaires externes ». Les extraits peptidiques de l'invention n'agissent donc pas comme un filtre ou un écran anti UVA ou anti UVB en ce sens que les cellules continuent à absorber ces rayons lumineux ; par contre, les extraits peptidiques de l'invention interviennent biologiquement au niveau des cellules, en limitant les effets néfastes des rayonnements tout en continuant à bénéficier des effets positifs.

Dans un mode de réalisation particulier, les extraits peptidiques de soja et de blé sont utilisés ensemble, par exemple dans un rapport en poids respectivement compris entre 80/20 et 20/80, avantageusement compris entre 70/30 et 30/70, de préférence égal à 60/40.

En pratique, les extraits peptidiques proviennent de graines de soja et de blé. Une hydrolyse enzymatique desdites graines par l'intermédiaire de peptidases permet de récupérer des peptides qui ont une taille moyenne de 700 Daltons (lorsque cette taille moyenne des peptides est atteinte, les peptidases sont alors inactivées).

Préférentiellement, l'extrait peptidique de soja est l'extrait identifié sous le numéro CAS 68607-88-5 de même que l'extrait peptidique de blé est l'extrait identifié sous le numéro CAS 70084-87-6.

En pratique, les extraits peptidiques précédemment décrits sont utilisés au sein d'une composition cosmétique comprenant un milieu physiologiquement acceptable.

On entend dans la présente demande par « milieu physiologiquement acceptable », un milieu compatible avec la peau, les lèvres, les cheveux et les muqueuses.

L'invention a donc pour objet une composition cosmétique et/ou dermatologique topique comprenant :

- des extraits peptidiques de soja et de blé représentant de 0,01 à 20 % du poids total de la composition,
- de la créatine,

ladite composition ne contenant pas le tripeptide synthétique GHK (glycyl-histidyl-lysine).

Avantageusement, les extraits peptidiques de soja et de blé représentent de 0,1 à 10 %, de préférence de 0,2 à 0,7% du poids total de la composition, très préférentiellement 0.4%.

Avantageusement, la créatine représente entre 0,1 et 2%, de préférence 0,5% en poids de la composition totale.

De manière caractéristique, la composition ne contient pas le tripeptide synthétique GHK (glycyl-histidyl-lysine ; INCI : Tripeptide-1).

De préférence, le rapport pondéral extrait peptidique de soja / extrait peptidique de blé est compris entre 80/20 et 20/80, avantageusement compris entre 70/30 et 30/70, de préférence égal à 60/40.

Comme déjà mentionné, le Demandeur a constaté que les extraits peptidiques de soja ou de blé, seuls ou en mélange permettaient de lutter contre l'altération des cellules de la peau, des lèvres, des cheveux et/ou des muqueuses provoquée par les UVA et UVB, à fortiori lorsque ces extraits étaient associés à des filtres solaires externes et/ou à d'autres constituants assurant la « protection interne » des cellules.

Le Demandeur a ainsi démontré que certains constituants exerçant une activité biologique in vivo sur les cellules de la peau, des lèvres, des cheveux et/ou des muqueuses soumises à un rayonnement UVA et UVB, agissaient de manière synergique avec les filtres solaires organiques ou minéraux permettant assurer d'une part «une protection interne », et d'autre part « une protection solaire externe » en réfléchissant ou absorbant la lumière. Il s'ensuit que le taux de filtres utilisé, peut être abaissé, diminuant de fait les risques précédemment évoqués.

En d'autres termes, dans un mode de réalisation particulier, la composition de l'invention comprend en outre au moins un filtre solaire choisi parmi les filtres minéraux, les filtres organiques ou leurs mélanges.

Tous les filtres minéraux connus en soi pour leur activité photoprotectrice externe par réfléchissement de la lumière sont utilisables dans la présente invention. Dans un mode de réalisation de l'invention, il s'agit d'oxydes métalliques, qui peuvent être notamment choisis parmi les oxydes de titane (dioxyde de titane amorphe ou cristallisé sous forme rutile et/ou anatase), les oxydes de zinc, les oxydes de fer, les oxydes de zirconium, les oxydes de cérium ou leurs mélanges.

Ces oxydes métalliques peuvent être sous forme de particules ayant une taille micrométrique ou nanométrique (nano-pigments). Dans un mode de réalisation particulier, les nano-pigments sont utilisés et leur taille moyenne est comprise par exemple entre 5 nm et 100 nm. Ces filtres minéraux peuvent être également enrobés.

Dans un mode de réalisation particulier de l'invention, on utilise des nano-oxydes de titane enrobés hydrophobes pouvant se présenter sous forme de charge solide ou sous forme de dispersion dans un milieu adapté à leur enrobage.

De même tous les filtres organiques UVA et UVB communément employés dans le domaine de la cosmétique, et connus pour leur activité photoprotectrice externe par absorption de la lumière sont utilisables dans la présente invention.

Comme filtres UVA, on peut citer par exemple les dérivés de dibenzoylméthane, les dérivés de benzophénone, les anthranilates, les dérivés de triazine, et les mélanges de ces filtres.

Comme filtres UVB, on peut citer par exemple les dérivés de l'acide salicylique, les dérivés de l'acide cinnamique, les dérivés de β,β'-diphénylacrylate liquide, les dérivés de l'acide p-aminobenzoïque, le 4-méthyl benzylidène camphre, l'acide 2-phénylbenzimidazole 5-sulfonique, les dérivés de 1,3,5-triazine, et les mélanges de ces filtres.

En fonction du résultat recherché, un mélange de filtres UVA et UVB peut être utilisé.

La composition de l'invention peut contenir de 0,5 à 50 % en poids de filtre solaire par rapport au poids total de la composition, bien que l'objectif soit qu'elle contienne une quantité de filtre moindre, de manière à diminuer les effets secondaires précédemment évoqués.

Dans un mode particulier de réalisation, la teneur en filtre solaire est comprise entre 1 et 20 %.

Dans un autre mode de réalisation, elle est comprise entre 2 % et 15 % par rapport au poids total de la composition, avantageusement entre 7 et 10%.

Le Demandeur a notamment démontré que la mise en oeuvre d'oxyde de titane, à raison de 2 à 5%, ou de butyl octyl salicylate, à raison de 7 à 10% en poids de la composition totale, donnait de bon résultats en terme de SPF (Sun Protecting Factor, indice permettant d'apprécier l'efficacité de protection d'un produit solaire, plus le SPF est élevé et meilleure sera la protection).

Selon une autre caractéristique de l'invention, la composition cosmétique et/ou dermatologique contient au moins un ou tous les constituants suivants exerçant une activité biologique in vivo sur les cellules de la peau, des lèvres, des cheveux et/ou des muqueuses soumises à une rayonnement UVA et UVB en présence ou non d'un filtre solaire, respectivement :

- un agent anti-radicalaire préservant les structures cellulaires, tel que par exemple la vitamine E et/ou ses dérivés liposolubles ou hydrosolubles, en en particulier ses isomères tocotrienol et/ou tocophérol, représentant par exemple entre 0,001 et 10%, avantageusement entre 0,02 et 2%, de préférence de l'ordre de 0,04% en poids de la composition totale,
- un agent limitant l'immunosuppression, tel que par exemple la vitamine PP, représentant par exemple entre 0,001 et 1%, avantageusement de 0,01% à 0,3% en poids de la composition totale,
- un agent protecteur de la protéine p53, tel que par exemple l'epigallocathéchine gallate (EGCG), représentant par exemple entre 0,001 et 0,1%, avantageusement de 0,005% à 0,05% en poids de la composition totale,
- un agent limitant l'action des ions fer impliqués dans la formation des radicaux libres, tel que par exemple l'EDTA, représentant par exemple entre 0,01 et 1%, avantageusement entre 0,2% et 0,5% en poids de la composition totale.

La composition de l'invention peut également contenir des adjuvants comme ceux habituellement utilisés dans le domaine de la cosmétique, tels que des actifs, des conservateurs, des antioxydants, des agents complexants, des solvants, des parfums, des charges, des bactéricides, des électrolytes, des absorbeurs d'odeur, des matières colorantes ou encore des vésicules lipidiques. Le choix de ces adjuvants, ainsi que leurs concentrations, doivent être déterminés de telle sorte qu'ils ne modifient pas les propriétés et les avantages recherchés pour la composition de la présente invention.

La composition de l'invention est destinée à une application topique et plus particulièrement à une application sur la peau, les lèvres, les cheveux et/ou les muqueuses.

La composition de l'invention peut se présenter sous toutes les formes galéniques normalement utilisées dans les domaines cosmétique et dermatologique, comme par exemple, mais de façon non limitative, sous la forme d'une solution aqueuse éventuellement gélifiée, d'une dispersion du type lotion pouvant être biphasée, d'une émulsion (H/E) ou inversement (E/H), plus ou moins fluide, ou d'une émulsion multiple comme par exemple une émulsion triple (E/H/E ou H/E/H), ou encore sous la forme d'une dispersion vésiculaire de type ionique (liposomes) et/ou non ionique. Ces compositions sont préparées selon les méthodes usuelles, connues de l'homme de l'art.

L'invention a également pour objet un procédé de traitement cosmétique pour prévenir l'altération des cellules de la peau, des lèvres, des cheveux et/ou des muqueuses provoquée par les UVA et UVB, caractérisé en ce qu'il consiste à appliquer sur la peau, les lèvres, les cheveux et/ou les muqueuses les compositions précédemment décrites.

L'invention a également pour objet une composition selon l'invention, en particulier une composition topique comprenant :

- des extraits peptidiques de soja et de blé,
- de la créatine,

ladite composition ne contenant pas le tripeptide synthétique GHK (gycyl-histidyl-lysine), pour son utilisation dans le traitement de l'altération des cellules de la peau, des lèvres, des cheveux et/ou des muqueuses provoquée par les UVA et les UVB.

Avantageusement, une composition selon l'invention pour cette utilisation est telle que décrite ci-dessus.

L'invention et les avantages qui en découlent ressortiront bien des exemples qui suivent.

## EFFICACITE PHOTOPROTECTRICE

### Exemple 1 : Efficacité photoprotectrice d'un mélange d'extraits peptidiques de soja et de blé utilisé seul, vis-à-vis des UVA

L'objectif de ce premier test est de démontrer que les extraits peptidiques de soja et de blé décrits dans la présente demande permettent à eux seuls de lutter contre les effets nocifs des rayonnements UVA en agissant sur le génome des cellules. Les essais sont conduits in vitro.

### Extrait désigné EV

Les extraits peptidiques sont obtenus par hydrolyse enzymatique de graines de soja ou de blé par l'intermédiaire de peptidases, les peptides obtenus ayant une taille moyenne de 700 Daltons. Les peptides de soja et de blé sont mis en solution à 10% dans un ratio 60/40, ladite solution étant ensuite diluée et testée à une concentration 1%. Les extraits utilisés correspondent aux numéros CAS 68607-88-5 (extrait peptidique de soja) et CAS 70084-87 (extrait peptidique de blé).

L'efficacité photoprotectrice du mélange d'extraits selon l'invention a été testée selon le plan d'expérience ci-après décrit.

La capacité de photoprotection interne de l'extrait végétal EV par son action sur le génome a été testée, contre les rayonnements UVA à 365 nm. L'évaluation a été réalisée par le test des comètes sur des primo-cultures de mélanocytes humains normaux (méthode décrite dans le brevet FR2774395 et dans la publication - Evaluation of Sunscreen protection in human melanocytes exposed to UVA or UVB irradiation using the alkaline cornet assay; Photochemistry and Photobiology, vol 74,Iss.3, P417 423- (2001)).

### Culture de mélanocytes

Les cultures de mélanocytes humains normaux (MHN) sont réalisées à partir de prépuces d'enfants et de nouveau-nés ayant un phimosis. Les mélanocytes obtenus à partir de fragments de peau sont placés dans le milieu MCDB 153 (Sigma St Louis, MO, USA) auquel ont été ajoutés 30 $\mu$g/ml d'extrait pituitaire bovin (BPE) (Life Technologies, Paisley, Angleterre), 2% de sérum de veau foetal (SVF) (Dominique Dutscher, Brumath, France), 16 nM de phorbol-12-myristate-13-acetate (Sigma), 5$\mu$g/ml d'insuline et 1,1 $\mu$M d'hydrocortisone (Sigma). Les cultures sont maintenues dans un incubateur à 37°C et sous atmosphère contenant 5% de $CO_2$. Des cultures pures de mélanocytes sont obtenues au bout de 2 à 3 semaines.

### Préparation des lames et irradiation des cellules

Après un traitement avec un mélange trypsine / EDTA (0,05% / 0,02%) pendant 2 à 3 minutes, les cellules sont récupérées par centrifugation et placées dans du tampon PBS sans $Ca^{2+}$ ni $Mg^{2+}$ (Sigma). Suivant une seconde centrifugation, les cellules (dont le nombre est compris entre 4,5 x $10^4$ et 5,0 x $10^4$) sont placées en suspension dans 0,5% d'agarose Low Melting Point, LMP (Sigma). Le mélange est directement déposé sur des lames de microscope qui sont recouvertes d'une pré-couche d'agarose (1,6%) séchée pendant une nuit à température ambiante et fraîchement précoatée avec une seconde couche d'agarose (0,8%). Une troisième couche d'agarose LMP est enfin déposée pour emprisonner les kératinocytes.

Les irradiations UVA sont générées par un irradiateur UV Bio-Sun (Vilbert Lourmat, Marne la Vallée). Cet appareil est équipé de lampes monochromatiques qui émettent à des longueurs d'onde de 365 nm. Les lampes délivrent une énergie calculée, à l'aide d'un radiomètre de type RMW-365/312 de 4.0 mW/cm$^2$ pour les UVA : les énergies délivrées sont alors de 0,8 J/cm$^2$. Les cellules sont irradiées par les UVA sur un bain de glace et le test des comètes est réalisé immédiatement après l'exposition.

Test des comètes (technique des lames sèches)

Le protocole du test des comètes est celui de De Méo et Coll (Méo M, Laget M, Castegnaro M, Duménil G. ; Genotoxic activity of potassium permanganate in acidic solutions ; Mutation Res. 1991; 260; 295-306) en incorporant la technique des « lames sèches » (Klaude M, Ericksson S, Nygren J, Annstrom G. The cornet assay: mechanism and technical considération. Mutation Res. 1996; 363; 89-96). Les lames sont placées, après les irradiations, dans un bain de lyse contenant 2,5 M de NaCl, 100 mM de $Na_2EDTA$, 10 mM de Tris-HCl pH 10, 1% de sodium sarcosinate, 1% de triton X-100 et 10% de DMSO. La lyse cellulaire s'effectue à 4°C pendant 60 min suivie par la dénaturation de l'ADN à température ambiante pendant 20 min dans une solution fortement alcaline comprenant 1 mM de $Na_2EDTA$ et 300 mM de NaOH à un pH supérieur à 13,0. Après une électrophorèse (25V, 300 mA) pendant 20 min, les lames sont neutralisées par le tampon Tris-HCl (0,4 M ; pH 7,4) et déshydratées dans de l'éthanol ou du méthanol absolu.

Observation microscopique et analyse d'images

Les lames sont colorées par une solution de bromure d'éthidium et observées à l'aide d'un microscope à fluorescence BH2-RFL (Olympus, Japon) équipé d'un filtre dichroïque 20BG-W (excitation : 515-560 nm ; émission : 590 nm) et d'un objectif Apo D-Plan 20x. L'analyse d'images s'effectue avec une caméra CCD monochrome haute sensibilité (Cohu 4912-5000) couplée à une carte d'acquisition Matrox IP-8. L'ensemble est piloté par le logiciel Fenestra Komet (Kinetic Imaging, Liverpool, RU, version 3.1).

Un total de 100 cellules par échantillon (50 cellules par lame) est analysé. Le paramètre utilisé est "Olive Tail Moment" (OTM qui est défini comme le produit du pourcentage d'ADN dans la queue de la comète par la longueur de celle-ci exprimée en $\mu m$). Pour chaque série d'expériences, un contrôle négatif (cellules non irradiées) et un contrôle positif (cellules irradiées sans écran) sont inclus.

La photoprotection interne a été mesurée après un traitement de 120 min à 37°C. La dose d'irradiation était de 0,8 $J/cm^2$ pour les rayonnements UVA (365 nm). Le test des comètes a été effectué immédiatement après irradiation. Les contrôles négatifs comprenaient des mélanocytes non traités et non irradiés et des mélanocytes traités par les deux mélanges et non irradiés. Le paramètre mesuré était le coefficient de protection génomique UVA ($CPG_{UVA}$).

Les résultats sont résumés dans le Tableau 1.

**Tableau 1 :** Protection interne de l'extrait végétal EV, 1%

| Lames N° | Condition | Moy±ET | Med | OTM$\chi^2$±ET | $CPG_{UVA}$ (%) |
|---|---|---|---|---|---|
| 1-2 | NI | 1,08±0,10 | 0,89 | 2,15±0,15 | 100% |
| 3-4 | UVA | 8,05±0,54 | 6,58 | 8,71±0,42 | 0% |
| 5-6 | EV/NI | 1,76±0,24 | 1,16 | 2,09±0,01 | - |
| 7-8 | EV/UVA | 5,86±0,38 | 4,95 | 7,49±0,26 | 17,7%* |

- NI : cellules non irradiées.
- UVA : cellules irradiées par des rayonnements UVA (0.8 $J/cm^2$).
- EV/NI : cellules traitées directement dans le milieu avec l'extrait végétal (1%) pendant deux heures à 37°C.
- EV/UVA : cellules traitées directement dans le milieu avec l'extrait végétal (1%) pendant deux heures à 37°C puis irradiées par des rayonnements UVA (0,8 $J/cm^2$).
- Probabilité avec :
- NS : non significatif ; * : $P < 0,05$ ; ** : $P < 0.01$ ; *** : $P < 0.001$
- Moy : moyenne des OTM
- Médiane OTM : valeur médiane de l'Olive Tail Moment
- $\chi^2$ OTM : paramètre calculé par régression des distributions des OTM selon une fonction $\chi^2$ (voir : Bauer et Coll (Bauer E, Recknagel RD, Fiedler U, Wollweber L, Bock C, Greulich KO ; the distribution of the tail moments in single cell electrophoresis (cornet assay) obeys a chi-square ($\chi^2$) not a gaussian distribution ; Mutation Res. 1998; 398: 101-110)).
- $CPG_{UVA}$ : coefficient de protection génomique UVA

Les coefficients de protection génomique ($CPG_{UVA}$) sont calculés avec la formule :

$$CPG = \left[ 1 - \frac{(\chi^2 OTM_e - \chi^2 OTM_{ce})}{(\chi^2 OTM_{C+} - \chi^2 OTM_{C-})} \right] x100$$

Avec $\chi^2 OTM_e$: $\chi^2$ OTM de l'échantillon ; $\chi^2 OTM_{ce}$: $\chi^2$ OTM du contrôle non irradié de l'échantillon; $\chi^2 OTM_{C-}$ : $\chi^2$ OTM du contrôle non irradié ; $\chi^2 OTM_{C+}$ : $\chi^2$ OTM du contrôle irradié.
Plus la valeur du CGP est grande, et plus la protection est grande. Par contre, c'est l'inverse pour l'OTM.

**Conclusion :**

Le CPG$_{UVA}$ de EV est de 17,7% ce qui démontre clairement que les extraits seuls ont une activité de protection du génome vis-à-vis des UVA.
Il est à noter que le test des comètes à été réalisé vis-à-vis des UVB mais il s'est avéré non concluant. Ce n'est pas pour autant que l'extrait EV serait totalement dépourvu d'efficacité vis-à-vis des UVB et il est au contraire démontré dans l'exemple 4 que in vivo, l'extrait EV a une activité vis-à-vis des UVB, probablement par le biais d'une autre voie que celle de la protection du génome.

**Exemple 2 : Efficacité photoprotectrice d'un mélange d'extraits peptidiques de soja et de blé utilisé en association avec des filtres, vis-à-vis des UVA**

Ce test vise à démontrer que l'extrait EV de l'exemple 1 conserve son action sur le génome vis-à-vis des UVA lorsqu'il est associé aux filtres UVA et UVB d'un lait solaire
L'efficacité photoprotectrice par le test des comètes du mélange d'extraits peptidiques de soja et de blé décrits dans la présente demande a été mesurée selon le même plan d'expérience que celui de l'exemple 1.
La capacité de photoprotection interne vis-à-vis des UVA à 365 nm de l'extrait végétal EV a été mesurée seul et en mélange avec un produit solaire de SPF 15 contenant une forte proportion de filtres UVA. (Lait Sun Trainer LST, réf : 03E.24EG31.1)
Les résultats sont résumés dans le Tableau 2.

**Tableau 2 :** Protection interne de l'extrait végétal EV, 1% et photoprotection du produit solaire LST contre les rayonnements UVA.

| Lames N° | Condition | Moy±ET | Med | OTM$\chi^2$±ET | CPG$_{UVA}$ (%) |
|---|---|---|---|---|---|
| 1-2 | NI | 1,08±0,10 | 0,89 | 2,15±0,15 | 100% |
| 3-4 | UVA | 8,05±0,54 | 6,58 | 8,71±0,42 | 0% |
| 5-6 | EV/NI | 1,76±0,24 | 1,16 | 2,09±0,01 | - |
| 7-8 | EV/UVA | 5,86±0,38 | 4,95 | 7,49±0,26 | 17,7%* |
| 9-10 | LST/UVA | 3,07±0,25 | 2,49 | 4,40±0,12 | 65,7%*** |
| 11-12 | LST/EV/UVA | 1,76±0,24 | 2,04 | 3,36±0,12 | 80,6%*** |
| - LST/UVA : 10 μl du Lait Sun Trainer SPF15 déposé sur une lame de quartz (5 cm x 5 cm) puis cellules irradiées par des rayonnements UVA (1,2 J/cm²). <br> - LST/EV/UVA : cellules traitées directement dans le milieu avec l'extrait végétal (1%) pendant 2 heures à 37°C puis irradiées par des UVA (0,8 J/cm²) à travers une lame de quartz (5 cm x 5 cm) sur laquelle est déposé un volume de 10 μl du Lait Sun Trainer. | | | | | |

**Conclusion :**

Le CPG$_{UVA}$ de EV est de 17,7%. Le CPG$_{UVA}$ de la photoprotection externe de LST est de 65,7%. Le cumul de la photoprotection interne de EV (1%) et de la photoprotection externe de LST est de 80,6%. Ces résultats montrent donc que l'effet photoprotecteur des extraits est maintenu même en présence des filtres UVA et UVB présents dans le Lait Sun Trainer.

**Exemple 3 : détermination du SPF in vitro et in vivo du mélange d'extraits EV de l'exemple 1 dilué à 4% au lieu de 1%**

Des tests ont été effectués pour démontrer l'efficacité des extraits peptidiques de soja et de blé décrits dans la présente demande lorsqu'il sont utilisés in vivo : détermination du SPF in vitro (réalisé selon la méthode décrite dans la publication du magazine Cosmetics ans Toiletries, vol 118, N° 10/Octobre 2003 : Détermination of the in vitro SPF) et détermination du SPF in vivo (réalisé selon la méthode COLIPA COLIPA (The European Cosmetic Toiletry and Perfumery Association), CTFA (Cosmetic, Toiletry & Fragrance Association of South Africa), JCIA (Japan Cosmetic Industry association) : International Sun protection factor (SPF) Test method. February 2003), avec un extrait végétal à 4%.

La formule testée (Base + EVà 4%) est la suivante et les résultats obtenus sont représentés dans le tableau 3.

| | |
|---|---|
| Dicapryl carbonate (Cetiol CC) | 17,00 % |
| Na acrylate/Na acryloyl-dimethyl taurate copolymer isohexadecane pollysorbate (Simulgel EG) | 3,00 % |
| Water | 2,40% |
| Peptide de blé | 0.15 % |
| Peptides de soja | 0.25 % |
| Water | qsp 100 % |
| Conservateurs (Phenonip) | 0,45 % |

**Tableau 3**

| Condition | SPF in vitro | SPF in vivo |
|---|---|---|
| Base + EVà 4% | 1.0 | 16.0 |

Le SPF ou Sun Protecting Factor, est un indice permettant d'apprécier l'efficacité de protection d'un produit solaire. Plus le SPF est élevé et meilleure sera la protection.

La valeur de 1 du SPF in vitro démontre que l'extrait n'a aucune activité anti UVA ou anti UVB sur la peau in vitro. En revanche, on obtient un effet biologique de l'extrait végétal in vivo qui est caractérisé par un SPF de 16. Il y a donc bien un effet de l'extrait vis-à-vis des cellules de la peau in vivo qui s'activent en réponse au rayonnement UVA/UVB.

**Exemple 4 protection solaire EV + filtre**

Des tests permettant de montrer l'efficacité de protection des extraits EV de l'exemple 1 dilués à 1% ou 4% ont été réalisés : détermination du SPF in vitro (réalisé selon la méthode décrite dans la publication du magazine Cosmetics ans Toiletries, vol 118, N° 10/Octobre 2003 : Détermination of the in vitro SPF) et détermination du SPF in vivo selon la méthode précédemment identifiée.

Le tableau 4 illustre les résultats obtenus pour des formulations comprenant l'extrait peptidique végétal EV et un oxyde minéral

**Tableau 4.**

| Condition Base + EV | SPF in vitro | SPF in vivo | Amélioration Apportée par l'extrait végétal |
|---|---|---|---|
| Oxyde de titane seul 3% | 6.1 | 6.4 | |
| EV à 4% | 1.0 | 16.0 | |
| Oxyde de titane 3% + Extrait EV à 1% | 6.4 | 10.2 | 59% |
| Oxyde de titane 3% EV à 4% | 5.8 | 25.5 | 298% |

Les résultats obtenus montrent qu'il y a une amélioration synergique de la protection solaire lorsqu'on associe l'extrait végétal avec l'oxyde minéral (le SPF de chacun des éléments pris indépendamment est respectivement de 6.4 et de 16.0, alors que le SPF obtenu avec l'ensemble est de 25.5, ce qui est supérieur à l'addition des SPF de deux éléments, soit 22.4).

Par ailleurs, sachant qu'un SPF de 6 correspond à environ 100% d'absorption des UVA s'il n'y a pas d'absorption UVB, et qu'un SPF supérieur caractérise forcément une filtration complémentaire des UVB, le tableau 4 montre que l'extrait décrit dans la présente demande a également un effet sur les UVB lorsqu'il est appliqué in vivo.

Exemple 5 : Composition selon l'invention à base de constituants agissant biologiquement (protection interne) et d'un filtre (protection externe)

La composition testée dans cet exemple comprend le noyau d'actifs suivant en % en poids:

| | |
|---|---|
| Octyl Butyl Salicylate (BHB) | 8% |
| Peptide de blé | 0.075% |
| Peptides de soja | 0.125% |
| Tocotrienol (oryza tocotrienol 90) | 0.3 % |
| Créatine | 0.5% |
| Vit PP | 0.3 % |
| Epigallocathéchine gallate | 0.05% |
| EDTA | 0.2% |
| C20-22 Alkyl Phosphate and C20-22 Alcohols (Sensanov WR) | 3,0% |
| Palmitate d'ethyl hexyl2 hexyle | 7,0% |
| Nacol 16-98 | 1,0% |
| BHT | 0,1% |
| Eau | 70,12 |
| NaOH | 0,08% |
| Hydroxyethyl Acrylate/Sodium Acryloldimethyl Taurate Copolymer and Squalane and Polysorbate 60 (Simulgel NS) | 3,0% |
| Eau | Qsp 100 |

Des tests ont été effectués pour démontrer l'efficacité de cette formule : détermination du SPF in vivo selon la même méthode que précédemment indiquée.

Le SPF de cette formulation est égal à 9,2 et ce, malgré la faible proportion d'extrait et de filtre. Cela montre donc l'effet de l'ensemble des actifs autres que le filtre, sur les cellules vis à vis des UVA et UVB.

## Revendications

1. Composition cosmétique et/ou dermatologique topique comprenant :

   - des extraits peptidiques de soja et de blé représentant de 0,01 à 20 % du poids total de la composition,
   - de la créatine,

   ladite composition ne contenant pas le tripeptide synthétique GHK (glycyl-histidyl-lysine).

2. Composition selon la revendication 1, *caractérisée* **en ce que** les extraits peptidiques de soja et de blé représentent de 0,1 à 10 % en poids, de préférence de 0,2 à 0,7% du poids total de la composition.

3. Composition selon la revendication 1 ou 2, *caractérisée* **en ce que** la créatine représente entre 0,1 et 2% en poids de la composition totale.

4. Composition selon l'une des revendications précédentes, *caractérisée* **en ce que** le rapport pondéral extrait peptidique de soja/extrait peptidique de blé est compris entre 80/20 et 20/80, avantageusement compris entre 70/30 et 30/70, de préférence égal à 60/40.

5. Composition selon l'une des revendications précédentes, *caractérisée* **en ce que** les extraits peptidiques sont obtenus par hydrolyse enzymatique de graines de soja ou de blé par l'intermédiaire de peptidases, les peptides obtenus ayant une taille moyenne de 700 Daltons.

6. Composition selon l'une des revendications précédentes, *caractérisée* **en ce que** la composition comprend en outre au moins un filtre solaire choisi parmi les filtres minéraux, les filtres organiques ou leurs mélanges.

7. Composition topique comprenant :

   - des extraits peptidiques de soja et de blé,
   - de la créatine,

   ladite composition ne contenant pas le tripeptide synthétique GHK (glycyl-histidyl-lysine), pour son utilisation dans le traitement de l'altération des cellules de la peau, des lèvres, des cheveux et/ou des muqueuses provoquée par les UVA et les UVB.

8. Composition pour son utilisation selon la revendication 7, *caractérisée* **en ce que** la composition est telle que définie dans les revendications 1 à 6.

**Patentansprüche**

1. Topische, kosmetische und/oder dermatologische Zubereitung, welche folgendes umfasst:

   - Peptidextrakte aus Soja und Weizen, welche 0,01 bis 20% Gewichtsprozent der Gesamtzubereitung ausmachen;
   - Kreatin,

   wobei die Zubereitung das synthetische Tripeptid GHK (Glykyl-Histidyl-Lysin) nicht enthält.

2. Zubereitung gemäß Anspruch 1, **dadurch gekennzeichnet, dass** die Peptidextrakte aus Soja und Weizen zwischen 0,1 bis 10% Gewichtsprozent, vorzugsweise zwischen 0,2 und 0,7% des Gesamtgewichts der Zubereitung ausmachen.

3. Zubereitung gemäß Anspruch 1 oder 2, **dadurch gekennzeichnet, dass** das Kreatin zwischen 0,1 und 2% des Gesamtgewichts der Zubereitung ausmacht.

4. Zubereitung gemäß einem der vorhergehenden Ansprüche, **dadurch gekennzeichnet, dass** das Gewichtsverhältnis Peptidextrakt aus Soja/ Peptidextrakt aus Weizen zwischen 80/20 und 20/80 liegt, vorteilhafterweise zwischen 70/30 und 30/70, am liebsten jedoch 60/40 entspricht.

5. Zubereitung gemäß einem der vorhergehenden Ansprüche, **dadurch gekennzeichnet, dass** die durch enzymatische Hydrolyse von Sojabohnen oder Weizenkörnern mittels Peptidasen erzielten Peptidextrakte aus Peptiden eine Durchschnittsgröße von 700 Daltons gewonnen wurden.

6. Zubereitung gemäß einem der vorhergehenden Ansprüche, **dadurch gekennzeichnet, dass** die Zubereitung außerdem mindestens einen Sonnenfilter aufweist, der unter mineralischen oder organischen Filtern oder einer Mischung daraus gewählt wird.

7. Topische Zubereitung, welche folgendes umfasst:

   - Peptidextrakte aus Soja und Weizen,
   - Kreatin,

   wobei die Zubereitung das synthetische Tripeptid GHK (Glykyl-Histidyl-Lysin) nicht enthält, zur Verwendung in der Behandlung von durch UVA und UVB verursachten Veränderungen der Hautzellen, der Lippen, der Haare und/oder der Schleimhäute.

8. Zubereitung zur Verwendung gemäß Anspruch 7, **dadurch gekennzeichnet, dass** die Zubereitung so ist, wie in den Ansprüchen 1 bis 6 definiert.

**Claims**

1. Topical cosmetic and/or dermatological composition comprising:

   - soybean and wheat peptide extracts representing from 0.01 to 20% of the total weight of the composition,
   - creatine,

   wherein said composition does not contain the synthetic tripeptide GHK (glycyl-histidyl-lysine).

2. Composition according to claim 1, **characterized in that** the soybean and wheat peptide extracts represent from 0.1 to 10% by weight, preferably 0.2 to 0.7% of the total weight of the composition.

3. Composition according to claim 1 or 2, **characterized in that** creatine represents between 0.1 and 2% of the total weight of the composition.

4. Composition according to one of the preceding claims, **characterized in that** the weight ratio of the soybean peptide extract /wheat peptide extract is between 80/20 and 20/80, advantageously between 70/30 and 30/70, preferably equal to 60/40.

5. Composition according to one of the preceding claims, **characterized in that** the peptide extracts are obtained by enzymatic hydrolysis of soybeans or wheat grains through peptidases, the peptides obtained having an average size of 700 Daltons.

6. Composition according to one of the preceding claims, **characterized in that** the composition further comprises at least one sunscreen selected from mineral filters, organic filters or mixtures thereof.

7. Topical composition comprising:

   - soybean and wheat peptide extracts,
   - creatine,

   wherein said composition does not contain the synthetic tripeptide GHK (glycyl-histidyl-lysine), for use in the treatment of cell damages of the skin, lips, hair and/or mucous membranes caused by UVA and UVB.

8. Composition for its use according to claim 7, **characterized in that** the composition is as defined in claims 1 to 6.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5571503 A
- FR 2775185
- WO 03068184 A
- WO 0064472 A
- WO 0217862 A
- EP 1618871 A

- WO 0245666 A
- US 6365656 B
- FR 2758721
- EP 1815843 A
- FR 2774395

**Littérature non-brevet citée dans la description**

- *CHEMICAL ABSTRACTS,* 68607-88-5
- *CHEMICAL ABSTRACTS,* 70084-87
- *Photochemistry and Photobiology,* 2001, vol. 74 (3), 417-423
- **MÉO M ; LAGET M ; CASTEGNARO M ; DUMÉNIL G.** Genotoxic activity of potassium permanganate in acidic solutions. *Mutation Res.,* 1991, vol. 260, 295-306
- **KLAUDE M ; ERICKSSON S ; NYGREN J ; ANNSTROM G.** The cornet assay: mechanism and technical considération. *Mutation Res.,* 1996, vol. 363, 89-96

- **BAUER E ; RECKNAGEL RD ; FIEDLER U ; WOLL-WEBER L ; BOCK C ; GREULICH KO.** the distribution of the tail moments in single cell electrophoresis (cornet assay) obeys a chi-square ($\chi 2$) not a gaussian distribution. *Mutation Res.,* 1998, vol. 398, 101-110
- *Cosmetics ans Toiletries,* 10 Octobre 2003, vol. 118